# EUROPÄISCHE PATENTANMELDUNG

(11) **EP 2 202 292 A1**
(43) Veröffentlichungstag der Anmeldung: **30.06.2010**
(21) Anmeldenummer: 09179726.6
(22) Anmeldetag: 17.12.2009
(51) Int. Cl.: C12M 1/107

(54) **Biogasanlage zur Methanisierung von Biomasse mit hohem Feststoffanteil**

(30) Priorität: 23.12.2008 DE 202008017049 U; 05.03.2009 DE 102009011868
(71) Anmelder: Bekon Energy Technologies GmbH & CO. KG, 85774 Unterföhring (DE)
(72) Erfinder: Lutz, Peter, 81927, München (DE)
(74) Vertreter: Winter, Brandl, Fürniss, Hübner, Röss, Kaiser, Polte - Partnerschaft

(57) **Zusammenfassung**

Eine Biogasanlage (10) zur Methanisierung von Biomasse mit hohem Feststoffanteil umfasst ein Faulbehältersystem mit einer Mehrzahl von gas- und flüssigkeitsdicht verschließbaren Faulbehältern (106), die jeweils eine Befüll- und Entnahmeöffnung zum Befüllten mit Biomasse und Entnehmen der Biomasse umfassen, eine Biogasableitungseinrichtung, einen Perkolatspeicher (20), eine Perkolat-Drainageeinrichtung (100) zum Ableiten von Perkolat aus der Mehrzahl von Faulbehältern (106) und Zuführen des Perkolats zu dem Perkolatspeicher (20), eine Perkolat-Verteileinrichtung (200) zum Verteilen des Perkolats aus dem Perkolatspeicher (20) über die Biomasse in der Mehrzahl von Faulbehältern (106), und eine Perkolat-Regelungseinrichtung zum Regeln des Perkolatspiegels in der Mehrzahl von Faulbehältern (106). Die Biogasanlage (10) ist **dadurch gekennzeichnet, dass** der Perkolatspeicher (20) einen ersten (22) und einen zweiten (24) Perkolatbehälter umfasst und mittels der Perkolat-Regelungseinrichtung das Zuführen des Perkolats zu und das Ableiten des Perkolats aus dem ersten (22) und / oder dem zweiten (24) Perkolatbehälter erfolgt.

## Beschreibung

Die vorliegende Erfindung betrifft eine Biogasanlage zur Methanisierung von Biomasse, insbesondere von Biomasse mit hohem Feststoffanteil, nach dem Oberbegriff des Anspruchs 1.

Der Begriff "Biomasse mit hohem Feststoffanteil" ist als Gegensatz zu flüssiger, pumpbarer Biomasse, wie sie in Nassgärverfahren eingesetzt wird, zu verstehen. "Biomasse mit hohem Feststoffanteil" ist daher als nicht pumpbare Biomasse zu verstehen.

Eine Biogasanlage nach dem Oberbegriff des Anspruchs 1 ist aus der WO 2007 / 096392 bekannt. Diese bekannte Biogasanlage umfasst eine Mehrzahl von gas- und flüssigkeitsdicht verschließbare Faulbehälter, die jeweils eine Perkolat-Ableitung mit einer Perkolat-Pumpe umfassen, die in einen gemeinsamen Perkolatspeicher münden. Über eine Perkolat-Rückleitung, in die eine Perkolat-Rückführpumpe geschaltet ist und die sich zu den einzelnen Faulbehältern verzweigt, kann Perkolat aus dem gemeinsamen Perkolatspeicher in die Faulbehälter zurückbefördert werden. Der Füllstand bzw. Perkolatpegel der einzelnen Faulbehälter wird durch jeweilige als Drucksensoren ausgebildete Füllstandssensoren erfasst.

Diese bekannte Biogasanlage hat den Nachteil, dass die Mehrzahl der Faulbehälter als einheitliches Gesamtsystem zu betrachten ist, da jeder Faulbehälter mit dem gleichen Perkolat beaufschlagt wird und in dem in jedem Faulbehälter im Wesentlichen die gleichen Umsetzungsprozesse stattfinden. Diese bekannte Biogasanlage ist somit verfahrenstechnisch einheitlich oder homogen und setzt sich hinsichtlich seiner Kapazität aus einer Mehrzahl von Teilsystemen (die einzelnen Faulbehälter) additiv zusammen.

Ausgehend von der Biogasanlage gemäß der WO 2007 / 096392 ist es Aufgabe der vorliegenden Erfindung, eine Biogasanlage mit einer Mehrzahl von Faulbehältern anzugeben die hinsichtlich der Umsetzungsprozesse in den einzelnen Faulbehältern flexibler ist und verfahrenstechnisch ein heterogenes Gesamtsystem bildet.

Diese Aufgabe wird durch die Merkmale des Anspruchs 1 gelöst.

Gemäß der vorliegenden Erfindung umfasst der Perkolatspeicher einen ersten und einen zweiten Perkolatbehälter, die jeweils über ein Leitungssystem mit jedem einzelnen der Mehrzahl von Faulbehältern verbindbar sind, wobei das Zuführen und Entnehmen des Perkolats zu den bzw. aus den Perkolatbehältern für jeden Faulbehälter separat regelbar ist. Damit liegen zwei getrennte Perkolatkreisläufe vor, so dass eine erste Teilmenge der Faulbehälter mit Perkolat aus dem ersten Perkolatbehälter und eine zweite Teilmenge der Faulbehälter mit Perkolat aus dem zweiten Perkolatbehälter beaufschlagt werden kann. In analoger Weise wird das Perkolat aus der ersten Teilmenge der Faulbehälter zurück in den ersten Perkolatspeicher und das Perkolat aus der zweiten Teilmenge der Faulbehälter zurück in den zweiten Perkolatbehälter geführt. Auf diese Weise ist es möglich, dass beispielsweise in der ersten Teilmenge von Faulbehältern Nawros (nachwachsende Rohstoffe) zu Biogas umgesetzt werden, während in der zweiten Teilmenge der Faulbehälter die organische Fraktion von Hausmüll umgesetzt wird. Würde man beide Arten von Biomasse mit dem gleichen Perkolat beaufschlagen, würde das Perkolat aus der belasteten organischen Fraktion des Hausmülls das "reinere" Perkolat der Nawros "verunreinigen". Die Produktion von Biogas würde in beiden Arten von Faulbehältern nicht optimal ablaufen, da sowohl die Nawros als auch die organische Fraktion des Hausmülls nicht mit dem für sie besonders geeigneten Perkolat beaufschlagt werden. Durch die voneinander getrennten Perkolatkreisläufe kann das Perkolat auf die jeweilige zu vergärende Biomasse - z. B. Nawros oder getrennt gesammelter Bioabfall und organische Hausmüllfraktion - angepasst werden. Damit werden quasi aus einer Biogasanlage verfahrenstechnisch zwei Biogasanlagen, die gemeinsam Biogas produzieren.

Gemäß Anspruch 12 können auch mehr als zwei getrennte Perkolatkreisläufe vorgesehen werden. Die Anzahl der getrennten Perkolatkreisläufe mit zugeordneten Perkolatbehältern legt die Anzahl der unterschiedlichen Biomassen fest, die in der erfindungsgemäßen Biogasanlage vergoren werden können.

Gemäß den Merkmalen des Anspruchs 2 verzweigen sich eine erste und eine zweite Drainage-Hauptleitung von dem ersten bzw. zweiten Perkolatbehälter kommend jeweils in Faulbehälter-Drainageleitungen, so dass jeder Faulbehälter mit dem ersten und / oder zweiten Perkolatbehälter verbindbar ist. Im Betrieb ist eine Trennung des ersten und zweiten Umsetzungsprozess dahingehend realisiert, dass alle Faulbehälter, in denen der erste Umsetzungsprozess stattfindet, mit dem ersten Perkolatbehälter verbunden sind, und alle Faulbehälter, in denen der zweite Umsetzungsprozess stattfindet, mit dem zweiten Perkolatbehälter verbunden sind. Welcher Prozess in welchem Faulbehälter stattfindet, kann erfindungsgemäß beliebig festgelegt werden.

Gemäß einer Ausgestaltung nach Anspruch 3 wird das Perkolat vor der Einleitung in den Perkolatspeicher gefiltert. Diese Filterung kann alternativ oder zusätzlich auch in den Leitungen vorgesehen sein, die das Perkolat von dem Perkolatspeicher zu den Faulbehältern zurückführt. Durch die Filterung ist gewährleistet, dass die Verteilanlage, z. B. eine Sprühanlage, durch in dem Perkolat vorhandene Schmutzpartikel, Schwebstoffe etc. nicht verstopft. Ferner können durch die Filterung Schwermetalle und dergleichen aus dem Perkolat entfernt werden.

Durch die Merkmale des Anspruchs 4 wird dahingehend eine konstruktive Vereinfachung erzielt, dass nicht von jeder der zwei Drainage-Hauptleitungen eine separate Leitung in jeden der Faulbehälter führt, sondern dass von jedem der Faulbehälter ein einzige als Drainage-Hauptabschnitt bezeichnete Leitung herausgeführt ist, die jeweils in eine erste und eine zweite als Drainage-Nebenabschnitt bezeichnete Leitung aufzweigen, die mit der ersten bzw. zweiten Drainage-Hauptleitung verbunden sind. Durch Steuerung von in den Drainage-Nebenabschnitten geschalteten Absperrventilen lässt sich jeder der Faulbehälter mit dem ersten und / oder mit dem zweiten Perkolatbehälter verbinden.

Die Merkmale des Anspruchs 5 erlauben eine beliebige räumliche Beziehung zwischen den Faulbehältern und dem Perkolatspeicher. Insbesondere kann der Perkolatspeicher an einer höheren Position als die Faulbehälter angeordnet sein, da das Perkolat mit Hilfe der Pumpen zu der höheren Position befördert werden kann.

Durch die Merkmale des Anspruchs 6 können die Faulbehälter unabhängig voneinander befüllt und geleert werden, da der Perkolatpegel jedes einzelnen Faulbehälters erfasst wird. Die durch die Perkolat-Regelungseinheit mit Hilfe des Füllstandssensors erfassten Daten (Höhe des Perkolatpegels) werden entsprechend den Bedingungen in den Faulbehältern als geeignetes Signal an die jeweiligen Pumpen gegeben, die durch Abpumpen von Perkolat den Perkolatpegel auf Sollniveau senken. Das Sollniveau kann z. B. auf der Grundlage der Befüllungsmenge des Faulbehälters oder der Zusammensetzung der Biomasse oder dergleichen bestimmt sein.

In dem erfindungsgemäßen Bioreaktor sind die Perkolat-Drainageeinrichtung und die Perkolat-Verteileinrichtung, die das Perkolat aus dem Perkolatspeicher entnimmt und über die Biomasse in den Faulbehältern verteilt, nahezu symmetrisch aufgebaut - Ansprüche 7 und 8.

Durch die vorteilhafte Ausgestaltung der Erfindung nach Anspruch 9 mit einer Mehrzahl von Verteil-Hauptabschnitten, die sich über die Querschnittsfläche des Faulbehälters verteilt in jeden der Faulbehälter erstrecken, wird eine gleichmäßige Verteilung des Perkolats über die gesamte Oberfläche der Biomasse in dem jeweiligen Faulbehälter erleichtert. Die gleichmäßige Verteilung des Perkolats kann weiterhin dadurch optimiert werden, dass für jeden Verteil-Hauptabschnitt - unabhängig von der Anzahl der Verteil-Hauptabschnitte - ein Prallteller an einer geeigneten Position angeordnet ist, auf den das aus diesem Verteil-Hauptabschnitt austretende Perkolat auftrifft und von ihm abprallend über der Biomasse versprüht wird.

Durch eine entsprechende Ventilanordnung wie sie in den Ansprüchen 8 und 11 definiert ist, kann äquivalent zu der Perkolat-Drainageeinrichtung jeder der Faulbehälter mit dem ersten und / oder dem zweiten Perkolatbehälter verbunden werden. Vorteilhafterweise erfolgt die Steuerung der Ventile automatisch derart, dass ein jeweiliger Faulbehälter über sein Drainage- und sein Perkolat-Verteileinrichtung nicht mit unterschiedlichen Perkolatbehältern verbunden ist.

Durch die Anordnung der Perkolatbehälter gemäß Anspruch 13 ist der Platzbedarf für den Perkolatspeicher gegenüber einer Anordnung der Perkolatbehälter nebeneinander reduziert, bei einer speziellen Anordnung gemäß Anspruch 14 zum Beispiel etwa auf die Hälfte. Damit verbunden ist außerdem ein vereinfachtes Leitungssystem, das die Perkolatbehälter mit den Faulbehältern verbindet. Darüber hinaus wirkt der äußere Perkolatbehälter als thermischer lsolierungsmantel des inneren Perkolatbehälters.

Die Merkmale des Anspruchs 15 ermöglichen ein einfaches und damit sicheres Befüllen der Faulbehälter durch geeignete landwirtschaftliche Maschinen.

Gemäß Anspruch 17 sind die Ventile pneumatisch betätigbar, so dass Explosionen der sich bei den Umsetzungsprozessen entwickelnden Gase, die durch Funkenbildung elektrisch betätigter Ventile ausgelöst werden können, ausgeschlossen.

Die übrigen Unteransprüche beziehen sich auf weitere vorteilhafte Ausgestaltungen der Erfindung.

Weitere Merkmale und Vorteile der vorliegenden Erfindung sind ersichtlich aus der nachfolgenden ausführlichen Beschreibung einer bevorzugten Ausführungsform mit Bezug auf die beiliegende Zeichnung. In der Zeichnungen sind:
Fig. 1 eine schematische Darstellung des Bioreaktors gemäß einer bevorzugten Ausführungsform der vorliegenden Erfindung;
Fig. 2 eine schematische Darstellung eines Schaltungsdetails der Perkolat-Drainageeinrichtung; und
Fig. 3 eine schematische Darstellung eines Schaltungsdetails der Perkolat-Verteileinrichtung.

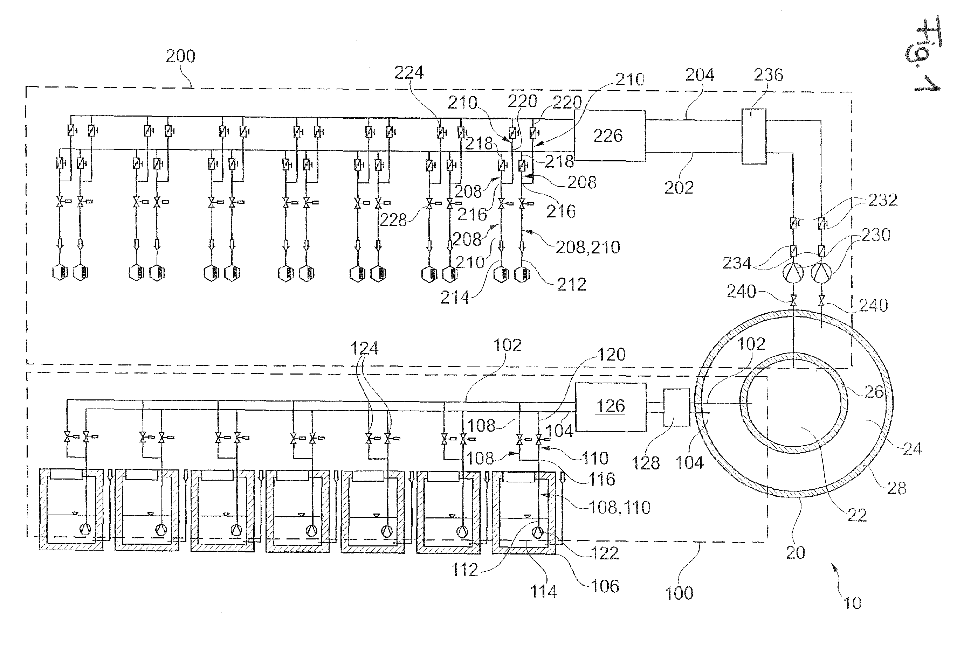

Wie es in Fig. 1 schematisch dargestellt ist, umfasst eine Biogasanlage 10 gemäß einer bevorzugten Ausführungsform einen Perkolatspeicher 20, eine Perkolat-Drainageeinrichtung 100, eine Perkolat-Verteileinrichtung 200 und eine Mehrzahl (in der Ausführungsform sieben) von Faulbehältern 106.

Der Perkolatspeicher 20 umfasst einen ersten, inneren, säulenförmigen Perkolatbehälter 22 und einen zweiten, äußeren, säulenförmigen Perkolatbehälter 24, wobei eine Trennwand 26, die die Perkolatbehälter 22, 24 voneinander trennt, und eine Außenwand 28 des Perkolatspeichers 20 im Schnitt betrachtet konzentrische Kreise bilden.

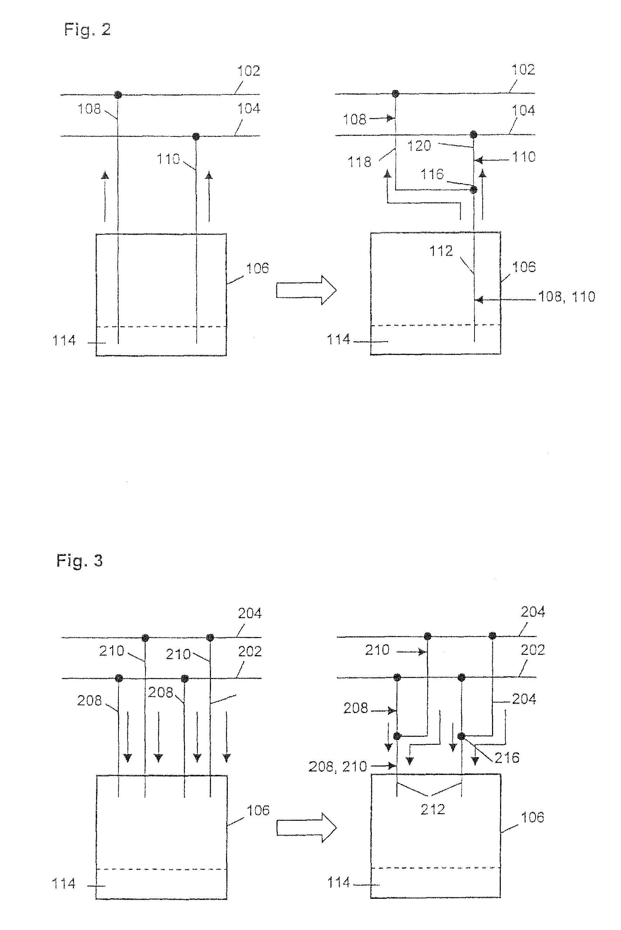

Die Perkolat-Drainageeinrichtung 100 umfasst eine erste Drainage-Hauptleitung 102 und eine zweite Drainage-Hauptleitung 104, die in den inneren 22 bzw. äußeren Perkolatbehälter 24 münden, und erste und zweite Faulbehälter-Drainageleitungen 108 bzw. 110, wobei jeder der Mehrzahl von Faulbehälter 106 über eine erste Faulbehälter-Drainageleitung 108 mit der ersten Drainage-Hauptleitung 102 und über eine zweite Faulbehälter-Drainageleitung 110 mit der zweiten Drainage-Hauptleitung 104 verbunden ist. Die erste und zweite Faulbehälter-Drainageleitung 108 bzw. 110 eines jeweiligen Faulbehälters 106 umfassen einen gemeinsamen Drainage-Hauptabschnitt 112, der sich von einem Perkolatbereich 114 innerhalb des Faulbehälters 106 bis zu einem Gabelungspunkt 116 außerhalb des Faulbehälters 106 erstreckt, und einen ersten bzw. zweiten Drainage-Nebenabschnitt 118 bzw. 120, die sich von dem Gabelungspunkt 116 zu der ersten bzw. zweiten Drainage-Hauptleitung 102 bzw. 104 erstrecken. Mit anderen Worten, die erste Faulbehälter-Drainageleitung 108 umfasst den Drainage-Hauptabschnitt 112 und den ersten Drainage-Nebenabschnitt 118, und die zweite Faulbehälter-Drainageleitung 110 umfasst den Drainage-Hauptabschnitt 112 und den zweiten Drainage-Nebenabschnitt 120, wie es (ohne Ventile) deutlicher in Fig. 2 gezeigt ist.

An jedem der Drainage-Hauptabschnitte 112 ist eine Pumpe 122 zum Befördern von Perkolat in den Perkolatspeicher 20 angeordnet, und in jedem Drainage-Nebenabschnitt 118, 120 befindet sich ein Absperrventil 124, durch das die Strömung durch diesen Abschnitt gesperrt oder freigegeben werden kann. Insbesondere kann das durch die Pumpe 122 beförderte Perkolat durch Sperren des Absperrventils 124 in dem ersten Drainage-Nebenabschnitt 118 und Öffnen des Absperrventils 124 in dem zweiten Drainage-Nebenabschnitt 120 über den Drainage-Hauptabschnitt 112, den zweiten Drainage-Nebenabschnitt 120 und die zweite Drainage-Hauptleitung 104 dem zweiten Perkolatbehälter 24 und durch Öffnen des Absperrventils 124 in dem ersten Drainage-Nebenabschnitt 118 und Sperren des Absperrventils 124 in dem zweiten Drainage-Nebenabschnitt 120 über den Drainage-Hauptabschnitt 112, den ersten Drainage-Nebenabschnitt 118 und die erste Drainage-Hauptleitung 102 dem ersten Perkolatbehälter 22 zugeführt werden.

Ferner kann durch entsprechende Schaltstellungen der den Faulbehältern 106 zugeordneten Absperrventile 124 das Perkolat des i-ten Faulbehälters 106 dem ersten Perkolatbehälter 22 und das Perkolat des j-ten Faulbehälters 106 dem zweiten Perkolatbehälter 24 zugeführt werden, wobei i, j ∈ {1,..., n} mit n = 7 in der Ausführungsform und i = j (bei identischen Umsetzungsprozessen in allen Faulbehältern 106, d. h. wenn keine Trennung erforderlich oder gewünscht ist) oder i ≠ j (bei verschiedenen Umsetzungsprozessen) ist. Zwischen den Faulbehältern 106 und dem Perkolatspeicher 20 ist ein erstes Filtersystem 126 in die erste und in die zweite Drainage-Hauptleitung 102 bzw. 104 geschaltet. Zwischen dem ersten Filtersystem und dem Perkolatspeicher 20 ist eine Durchflussmessvorrichtung 128 geschaltet, die den Perkolatdurchsatz in der ersten und / oder der zweiten Drainage-Hauptleitung 102 bzw. 104 erfasst. Auf der Grundlage der Erfassungsdaten und weiterer Daten können die Ventile optimal angesteuert werden.

Als Pendant zu der ersten und zweiten Drainage-Hauptleitung 102 bzw. 104 der Perkolat-Drainageeinrichtung 100 umfasst die Perkolat-Verteileinrichtung 200 eine erste und eine zweite Verteil-Hauptleitung 202 bzw. 204 (vgl. auch Fig. 3), die in den ersten bzw. dem zweiten Perkolatbehälter 22 bzw. 24 münden. Jeder der Faulbehälter 106 ist über zwei erste Faulbehälter-Verteilleitungen 208 mit der ersten Verteil-Hauptleitung 202 und über zwei zweite Faulbehälter-Verteilleitung 210 mit der zweiten Verteil-Hauptleitung 204 verbunden (siehe Fig. 3). Eine der ersten Faulbehälter-Verteilleitungen 208 und eine der zweiten Faulbehälter-Verteilleitungen 210 sind so miteinander verbunden, dass sie einen gemeinsamen Verteil-Hauptabschnitt 212, der sich von einem Gasbereich des Faulbehälters 106 bis zu einem Verbindungspunkt 216 erstreckt, und einen ersten bzw. zweiten Verteil-Nebenabschnitt 218 bzw. 220, die sich von dem Verbindungspunkt 216 zu der ersten bzw. zweiten Verteil-Hauptleitung 202 bzw. 204 erstrecken, umfassen. Die zweite der ersten Faulbehälter-Verteilleitungen 208 und die zweite der zweiten Faulbehälter-Verteilleitungen 210 sind entsprechend miteinander verbunden. Mit anderen Worten, die ersten Faulbehälter-Verteilleitungen 208 umfassen jeweils einen Verteil-Hauptabschnitt 212 und einen Verteil-Nebenabschnitt 218, und die zweiten Faulbehälter-Verteilleitungen 210 umfassen jeweils einen Verteil-Hauptabschnitt 214 und einen Verteil-Nebenabschnitt 220.

In den Verteil-Nebenabschnitten 218, 220 ist jeweils ein Durchflusssteuerungsventil 224 angeordnet, und in den Verteil-Hauptabschnitten 212 ist jeweils ein Absperrventil 228 angeordnet. Zwischen die Faulbehälter 106 und den Perkolatspeicher 20 ist ein Filtersystem 226 in die Verteil-Hauptleitungen 202, 204 geschaltet. Ferner sind in den Verteil-Hauptleitungen 202, 204 Pumpen 230 zum Befördern des Perkolats aus dem Perkolatspeicher 20 in die jeweiligen Faulbehälter 106 sowie Durchflusssteuerventile 232 und Sperrventile 234 angeordnet. Analog zu der Perkolat-Drainageeinrichtung 100 kann durch geeignete Stellungen der Ventile 224 und 228 das Perkolat aus dem ersten und zweiten Perkolatbehälter 22 bzw. 24 zusammen oder das Perkolat aus nur einem von dem ersten und dem zweiten Perkolatbehälter 22 bzw. 24 jedem der Faulbehälter 106 unabhängig voneinander zugeführt werden. Mit anderen Worten, durch die Ventile 224, 228 kann für jeden der Faulbehälter 106 unabhängig von den weiteren der Faulbehälter 106 eine beliebige Perkolatmischung aus dem ersten und / oder zweiten Perkolatbehälter 22 bzw. 24 zugeführt werden, die über die Verteil-Hauptabschnitte 212 über der Biomasse verteilt wird. Entsprechend der Perkolat-Drainageeinrichtung 100 ist zwischen dem Filtersystem 226 und dem Perkolatspeicher 20 eine Durchflussmessvorrichtung geschaltet, um den Perkolatdurchsatz in der ersten und / oder zweiten Verteil-Hauptleitung 202 bzw. 204 zu erfassen.

Die Faulbehälter 106 umfassen jeweils eine Biogasableitung (nicht gezeigt), über die das in dem jeweiligen Faulbehälter 106 erzeugte Biogas abgeführt wird.

Gemäß der Ausführungsform werden alle Ventile und Pumpen von einer Perkolat-Regelungseinheit (nicht gezeigt) angesteuert. Die Regelung erfolgt zum Beispiel auf der Grundlage der Erfassungswerte des Perkolatpegels, der zum Beispiel über einen Drucksensor erfasst werden kann, der Temperatur der Biomasse, der Zusammensetzung des abgeführten Biogases etc.

Obgleich die vorliegende Erfindung bezüglich der bevorzugten Ausführungsformen offenbart worden ist, um ein besseres Verständnis von diesen zu ermöglichen, sollte wahrgenommen werden, dass die Erfindung auf verschiedene Weisen verwirklicht werden kann, ohne den Umfang der Erfindung zu verlassen. Deshalb sollte die Erfindung derart verstanden werden, dass sie alle möglichen Ausführungsformen und Ausgestaltungen zu den gezeigten Ausführungsformen beinhaltet, die realisiert werden können, ohne den Umfang der Erfindung zu verlassen, wie er in den beigefügten Ansprüchen dargelegt ist.

### Bezugszeichenliste

- 10: Biogasanlage
- 20: Perkolatspeicher
- 22: Erster, innerer Perkolatbehälter
- 24: Zweiter, äußerer Perkolatbehälter
- 26: Trennwand zwischen 22 und 24
- 28: Außenwand von 20

- 100: Perkolat-Drainageeinrichtung
- 102: Erste Drainage-Hauptleitung
- 104: Zweite Drainage-Hauptleitung
- 106: Faulbehälter
- 108: Erste Faulbehälter-Drainageleitung
- 110: Zweite Faulbehälter-Drainageleitung
- 112: Drainage-Hauptabschnitt
- 114: Perkolatbereich
- 116: Gabelungspunkt zwischen 112 und 116, 118
- 118: Erster Drainage-Nebenabschnitt
- 120: Zweiter Drainage-Nebenabschnitt
- 122: Pumpe an 112
- 124: Absperrventil in 118, 120
- 126: Filtersystem von 100
- 128: Durchflussmessvorrichtung

- 200: Perkolat-Verteileinrichtung
- 202: Erste Verteil-Hauptleitung
- 204: Zweite Vorteil-Hauptleitung
- 208: Erste Faulbehälter-Verteilleitung
- 210: Zweite Faulbehälter-Verteilleitung
- 212: Verteil-Hauptabschnitt
- 216: Verbindungspunkt zwischen 214 und 218, 220

- 218: Erster Verteil-Nebenabschnitt
- 220: Zweiter Verteil-Nebenabschnitt
- 224: Durchflusssteuerventile in 218, 220
- 228: Absperrventile in 212
- 226: Filtersystem in 202, 204
- 230: Pumpen in 202, 204
- 232: Durchflusssteuerventile in 202, 204
- 234: Absperrventile in 202, 204
- 236: Durchflussmessvorrichtung

## Patentansprüche

1. Biogasanlage (10) zur Methanisierung von Biomasse mit hohem Feststoffanteil, mit:
- einem Faulbehältersystem mit einer Mehrzahl von gas- und flüssigkeitsdicht verschließbaren Faulbehältern (106), die jeweils eine Befüll- und Entnahmeöffnung zum Befüllen mit Biomasse und Entnehmen der Biomasse umfassen;
- einer Biogasableitungseinrichtung;
- einem Perkolatspeicher (20);
- einer Perkolat-Drainageeinrichtung (100) zum Ableiten von Perkolat aus der Mehrzahl von Faulbehältern (106) und Zuführen des Perkolats zu dem Perkolatspeicher (20);
- einer Perkolat-Verteileinrichtung (200) zum Verteilen des Perkolats aus dem Perkolatspeicher (20) über die Biomasse in der Mehrzahl von Faulbehältern (106); und
- einer Perkolat-Regelungseinrichtung zum Regeln des Perkolatspiegels in der Mehrzahl von Faulbehältern (106);
**dadurch gekennzeichnet, dass**
- der Perkolatspeicher (20) einen ersten (22) und einen zweiten (24) Perkolatbehälter umfasst; und
- mittels der Perkolat-Regelungseinrichtung das Zuführen des Perkolats zu und das Ableiten des Perkolats aus dem ersten (22) und / oder dem zweiten (24) Perkolatbehälter erfolgt.

2. Biogasanlage (10) nach Anspruch 1, **dadurch gekennzeichnet, dass**:
- die Perkolat-Drainageeinrichtung (100) eine erste (102) und eine zweite (104) Drainage-Hauptleitung umfasst, die den ersten (22) bzw. den zweiten (24) Perkolatbehälter mit den Faulbehältern (106) verbinden;
- die erste (102) und die zweite (104) Drainage-Hauptleitung in erste (108) bzw. zweite (110) Faulbehälter-Drainageleitungen verzweigen, wobei jedem der Mehrzahl von Faulbehältern (106) eine der ersten (108) und eine der zweiten (110) Faulbehälter-Drainageleitungen zugeordnet sind, die in einen Perkolatbereich des Faulbehälters (106) münden.

3. Biogasanlage (10) nach Anspruch 2, **dadurch gekennzeichnet, dass** zwischen dem Faulbehältersystem und dem Perkolatspeicher (20) ein Drainage-Filtersystem (126) in die erste (102) und / oder die zweite (104) Drainage-Hauptleitung geschaltet ist.

4. Biogasanlage (10) nach Anspruch 2 oder 3, **dadurch gekennzeichnet, dass** die erste (108) und die zweite (110) Faulbehälter-Drainageleitung eines Faulbehälters (106) an einem Gabelungspunkt (116) in einen mit der ersten Drainage-Hauptleitung (102) verbundenen ersten Drainage-Nebenabschnitt (118) bzw. einen mit der zweiten Drainage-Hauptleitung (104) verbundenen zweiten Drainage-Nebenabschnitt (120) und einen gemeinsamen, sich in den Faulbehälter (106) erstreckenden Drainage-Hauptabschnitt (112) unterteilt sind und in jedem der Drainage-Nebenabschnitte (118, 120) ein Absperrventil (124) angeordnet ist.

5. Biogasanlage (10) nach einem der Ansprüche 2 bis 4, **dadurch gekennzeichnet, dass** jede Faulbehälter-Drainageleitung (108, 110) eine Pumpe (122) zum Fördern von Perkolat in den Perkolatspeicher (20) umfasst.

6. Biogasanlage (10) nach einem der Ansprüche 1 bis 5, **dadurch gekennzeichnet, dass** jeder Faulbehälter (106) einen Füllstandssensor zur Bestimmung eines Perkolatpegels umfasst, der mit der Perkolat-Regelungseinheit verbunden ist.

7. Biogasanlage nach einem der Ansprüche 1 bis 6, **dadurch gekennzeichnet, dass**:
- die Perkolat-Verteileinrichtung (200) eine erste (202) und eine zweite (204) Verteil-Hauptleitung umfasst, die den ersten (22) bzw. zweiten (24) Perkolatbehälter mit den Faulbehältern (106) verbinden;
- die erste (202) und die zweite (204) Verteil-Hauptleitung in erste (208) bzw. zweite (210) Faulbehälter-Verteilleitungen verzweigen, wobei jedem der Mehrzahl von Faulbehältern (106) eine der ersten (208) und eine der zweiten (210) Faulbehälter-Verteilleitungen zugeordnet ist, die in einen Gasbereich des Faulbehälters (106) münden.

8. Biogasanlage (10) nach Anspruch 7, **dadurch gekennzeichnet, dass** die erste (208) und die zweite (210) Faulbehälter-Verteilleitung eines Faulbehälters (106) an einem Verbindungspunkt (216) in einen ersten (218) bzw. einen zweiten (220) Verteil-Nebenabschnitt und einen gemeinsamen, sich in den Faulbehälter (106) erstreckenden Verteil-Hauptabschnitt (212) unterteilt sind und in jedem der Verteil-Nebenabschnitte (218, 220) ein Durchflusssteuerungsventil (224) angeordnet ist.

9. Biogasanlage (10) nach Anspruch 8, **dadurch gekennzeichnet, dass** sich in jeden der Faulbehälter (106) eine Mehrzahl von Verteil-Hauptabschnitte (212) erstrecken, die jeweils an dem Verbindungspunkt (216) in den mit der ersten Verteil-Hauptleitung (202) verbundenen ersten Verteil-Nebenabschnitt (218) und den mit der zweiten Verteil-Hauptleitung (204) verbundenen zweiten Verteil-Nebenabschnitt (220) aufzweigen.

10. Biogasanlage (10) nach einem der vorhergehenden Ansprüche 7 bis 9, **dadurch gekennzeichnet, dass** zwischen dem Faulbehältersystem und dem Perkolatspeicher (20) ein Verteil-Filtersystem (226) in die erste (202) und / oder zweite (204) Verteil-Hauptleitung geschaltet ist.

11. Biogasanlage (10) nach einem der vorhergehenden Ansprüche 7 bis 10, **dadurch gekennzeichnet, dass** in jedem der Verteil-Hauptabschnitte (212) ein Absperrventil (228) angeordnet ist.

12. Biogasanlage nach einem der vorherigen Ansprüche, **dadurch gekennzeichnet, dass** wenigstens drei voneinander getrennte Perkolatkreisläufe mit wenigstens einem dritten Perkolatbehälter, wenigstens einer dritten Verteil-Hauptleitung und wenigstens einer dritten Drainage-Hauptleitung vorgesehen sind.

13. Biogasanlage (10) nach einem der vorhergehenden Ansprüche, **dadurch gekennzeichnet, dass** die einzelnen Perkolatbehälter (22, 24) ineinander angeordnet sind.

14. Biogasanlage (10) nach einem der vorhergehenden Ansprüche, **dadurch gekennzeichnet, dass** die einzelnen Perkolatbehälter (22, 24) in Form konzentrischer Rohre ausgebildet sind.

15. Biogasanlage (10) nach einem der vorhergehenden Ansprüche, **dadurch gekennzeichnet, dass** die Befüll- und Entnahmeöffnung eine vorzugsweise hydraulisch betätigbare Klappe ist, die ebenerdig den Faulbehälter (106) verschließt.

16. Biogasanlage (10) nach einem der vorhergehenden Ansprüche, **dadurch gekennzeichnet, dass** die Mehrzahl von Faulbehältern (106) jeweils eine Rückhaltevorrichtung umfasst, die in Befüllrichtung hinter der Klappe derart in dem Faulbehälter (106) angeordnet ist, dass eingefüllte Biomasse sich wenigstens teilweise an der Rückhaltevorrichtung abstützt.

17. Biogasanlage (10) nach einem der vorhergehenden Ansprüche 4 bis 16, **dadurch gekennzeichnet, dass** die Ventile (124, 224, 228) pneumatisch betätigbare Ventile sind.
